# EUROPÄISCHE PATENTSCHRIFT

(11) **EP 0 936 208 B1**
(45) Veröffentlichungstag und Bekanntmachung des Hinweises auf die Patenterteilung: **23.04.2003**
(21) Anmeldenummer: 99102630.3
(22) Anmeldetag: 11.02.1999
(51) Int. Cl.: C07C 45/65, C07C 49/603, C07C 49/593, C07C 49/607, C07C 49/597, C25B 3/02

(54) **Verfahren zur Herstellung von 2-Cycloalkenonen**
Process for the preparation of 2-cycloalkenones
Procédé pour la préparation de 2-cycloalcénones

(30) Priorität: 12.02.1998 DE 19805778
(43) Veröffentlichungstag der Anmeldung: 18.08.1999
(73) Patentinhaber: BASF AKTIENGESELLSCHAFT, 67056 Ludwigshafen (DE)
(72) Erfinder: Eiermann, Matthias,, 67117 Limburgerhof (DE); Ebel, Klaus,, 68623 Lampertheim (DE); Botzem, Jörg, 67117 Limburgerhof (DE)
(74) Vertreter: Kinzebach, Werner, Dr.

(56) Entgegenhaltungen:
- EP-A- 0 460 451
- DE-A- 2 150 294
- FR-A- 1 567 440
- US-A- 3 523 125

## Beschreibung

Die vorliegende Erfindung betrifft ein Verfahren zur Herstellung von 2-Cycloalkenonen ausgehend von 2-Alkoxycycloalkanonen oder 2-Hydroxycycloalkanonketalen durch eine Eliminierungsreaktion in der Gasphase an einem sauren heterogenen Katalysator.

2-Cycloalkenone sind vielseitig einsetzbare Ausgangs- oder Zwischenprodukte, u. a. zur Herstellung pharmakologisch wirksamer Substanzen. Als Beispiele seien die Herstellung von Carbazolen und Carbazol-Derivaten als veterinärmedizinisch relevante Antiphlogistika gemäß EP-A-008 446 und die Herstellung von Prostaglandinen ausgehend von 2-Cyclopentenonen genannt.

Gleichwohl weisen die bekannten Verfahren zur technischen Herstellung von 2-Cycloalkenonen erhebliche Nachteile auf. Bekannte Verfahren, die auf leicht und billig in technischen Mengen zugänglichen Edukten basieren, sind technisch aufwendig oder führen zu schwer verwertbaren Nebenprodukten. Beispiele hierfür sind die Birch-Reduktion von Anisol, ein aufwendiges, mehrstufiges Verfahren unter Einsatz von Natrium und flüssigem Ammoniak, das nur mäßige Ausbeuten liefert und zudem auf die Herstellung von 2-Cyclohexenon beschränkt ist, sowie die Oxidation von Cycloalkenen mit Peroxiden (J. Mlochowski, S. B. Said, Polish J. Chem. 1997, 71, S. 149), die auf Grund geringer Selektivität zu einer Vielzahl schwer abtrennbarer Nebenprodukte führt.

Ein technisches Verfahren ist in der DE-A-21 50 294 offenbart. Darin wird aus 2-Halogencyclohexanon Halogenwasserstoff bzw. aus 2-Acyloxycyclohexanon die jeweilige Carbonsäure thermisch eliminiert. Die gravierenden Nachteile dieses Verfahrens sollen am Beispiel der Darstellung von 2-Cyclohexenon aus 2-Chlorcyclohexanon aufgezeigt werden. Hier muss das Cyclohexanon zuerst ketalisiert, dann chloriert, dehydrochloriert und anschließend einer sauren Ketalspaltung unterworfen werden. Darüberhinaus entstehen bei der Chlorierung Regioisomere und Mehrfachchlorierungsprodukte, die abgetrennt werden müssen oder zur Bildung von Produktgemischen von 2- und 3-Cyclohexenonen führen können, die wiederum aufwendig getrennt oder isomerisiert werden müssen.

Der vorliegenden Erfindung liegt daher die Aufgabe zu Grunde, ein breit anwendbares Verfahren zur Herstellung von 2-Cycloalkenonen bereitzustellen, das auf einfach und in technischen Mengen zugänglichen Edukten basiert und in möglichst wenigen Stufen die gewünschten Cycloalkenone liefert.

Es wurde nun überraschenderweise gefunden, dass sich aus den leicht zugänglichen 2-Alkoxycycloalkanonen bzw. 2-Hydroxycycloalkanonketalen durch sauer heterogen katalysierte Eliminierung die gewünschten 2-Cycloalkenone in einfacher Weise, in einer Stufe und in guten Ausbeuten herstellen lassen.

Gegenstand der vorliegenden Erfindung ist daher ein Verfahren zur Herstellung von 2-Cycloalkenonen der Formel I, 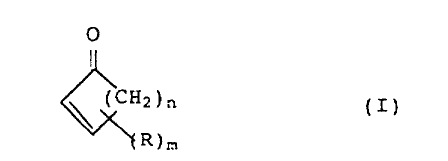 das dadurch gekennzeichnet ist, dass man ein 2-Alkoxycycloalkanon der Formel II 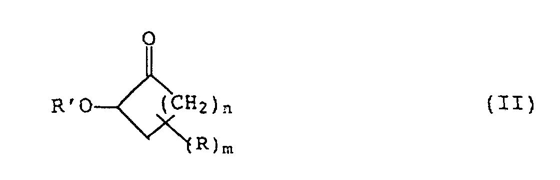 oder ein 2-Hydroxycycloalkanonketal der Formel III, 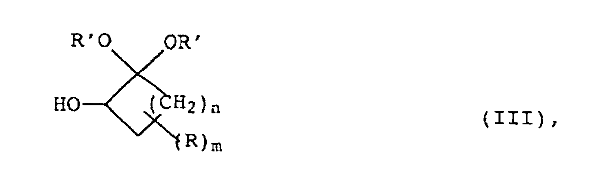 wobei
- n: für eine ganze Zahl von 0 bis 20, bevorzugt für 1 bis 8, besonders bevorzugt für 2 und 3, steht,
- m: für 0, 1, 2 oder 3 steht,
- die Reste R: unabhängig voneinander Alkyl, Alkylaryl, Aryl, Arylalkyl, Alkenyl oder Alkinyl stehen, wobei die Arylreste einen oder zwei Substituenten aufweisen können, die unabhängig voneinander ausgewählt sind unter Alkyl, Hydroxy oder Alkoxy,
- die Reste R': unabhängig voneinander Alkyl oder Alkenyl bedeuten oder in Formel III gemeinsam für C₁-C₅-Alkylen oder C₂-C₅-Alkenylen stehen können,
oder ein Gemisch aus den Verbindungen der Formeln II und III an einem sauren heterogenen Katalysator einer Eliminierungsreaktion in der Gasphase unterwirft.

Alkyl (auch in Alkylaryl, Alkoxy etc.) steht für geradkettige oder verzweigte Alkylgruppen, die vorzugsweise 1 bis 12, insbesondere 1 bis 6 Kohlenstoffatome aufweisen. Beispiele sind Methyl, Ethyl, n-Propyl, iso-Propyl, n-Butyl, sek.-Butyl, isoButyl, t-Butyl, n-Hexyl, 2-Ethylhexyl und n-Dodecyl.

Aryl steht insbesondere für Phenyl oder Naphthyl.

Alkenyl steht für geradkettige oder verzweigte Alkenylgruppen mit vorzugsweise 2 bis 6 Kohlenstoffatomen. Beispiele sind Allyl, Methallyl, 2-Buten-1-yl, und 3-Buten-1-yl.

Alkinyl steht für geradkettige oder verzweigte Alkinylgruppen mit vorzugsweise 2 bis 6 Kohlenstoffatomen. Beispiele sind 2-Propin-1-yl, 2-Butin-1-yl und 3-Butin-1-yl.

Vorzugsweise steht in den obigen Formeln:
- R: für H (m = 0), Alkyl oder Aryl, das gegebenenfalls durch einen oder zwei Alkyl-, Hydroxy- oder Alkoxyreste substituiert ist. Besonders bevorzugt steht R für H;
- m: für 0 oder 1;
- n: für 2 bis 5, insbesondere 2 bis 4; und
- R': für Alkyl oder C₁-C₅-Alkylen.

Besonders bevorzugt ist die Herstellung von 2-Cyclopentenon und 2-Cyclohexenon aus 2-Hydroxycyclopentanon- bzw. 2-Hydroxycyclohexanon-C₁-C₆-dialkylketal oder C₁-C₆-Alkylcyclopentanon bzw. C₁-C₆-Alkylcyclohexanon.

Erfindungsgemäß bevorzugt ist der Einsatz eines sauren heterogenen Katalysators, der wenigstens ein Oxid oder Phosphat, oder eine Mischung von Oxiden und Phosphaten, oder Mischoxide von Elementen der Gruppen 3 bis 6 und 13 bis 15 des Periodensystems, gezählt nach der derzeit gültigen IUPAC-Empfehlung, enthält. Insbesondere enthält der saure heterogene Katalysator Oxide des Titans, Zirkoniums, Aluminiums, Siliciums, und/oder Phosphate des Zirkoniums, Lanthans und Cers, besonders bevorzugt TiO₂, ZrO₂, Al₂O₃, SiO₂ und LaPO₄.

Vorzugsweise ist der saure heterogene Katalysator zusätzlich mit einer oder mehreren sauren Verbindungen imprägniert. Geeignete saure Verbindungen umfassen Mineralsäuren wie Phosphorsäure, Salpetersäure, Schwefelsäure, Salzsäure, ihre kondensierten Formen wie Pyrophosphorsäure, Pyroschwefelsäure, und ihre Metall- oder Ammoniumsalze. Als Metalle sind die Elemente der Gruppen 1 bis 14 des Periodensystems, gezählt gemäß derzeitiger IUPAC-Empfehlung, insbesondere jedoch die Alkali- und Erdalkalimetalle, zu verstehen. Der Begriff Ammonium steht für die protonierte oder alkylierte Form des Ammoniaks oder für ein Mono-, Di- oder Trialkylamin, wobei die Alkylreste unabhängig voneinander 1 bis 20, insbesondere 1 bis 6, Kohlenstoffatome aufweisen können. Gegebenenfalls kann der saure heterogene Katalysator auf einen Träger aufgebracht sein. Geeignete Träger sind Siliciumdioxid (Kieselgel), Aluminiumoxid, Siliciumcarbid, Siliciumnitrit, Graphit, Zeolithe und andere unter den Reaktionsbedingungen inerte Feststoffe.

Die Menge an saurer Verbindung liegt im Allgemeinen im Bereich von 1 bis 70 Gew.-%, vorzugsweise 5 bis 50 Gew.-%, bezogen auf das Oxid oder Phosphat.

Das erfindungsgemäße Verfahren wird im Allgemeinen kontinuierlich, insbesondere in einem rohrförmigen Reaktor, durchgeführt. Der Katalysator kommt dabei im Allgemeinen in stückiger Form, z. B. in Form von Strangpreßlingen, Tabletten oder Kugeln, zur Anwendung. Bevorzugt ist die Verwendung als Festbett.

Auch die erfindungsgemäß verwendeten sauren heterogenen Katalysatoren unterliegen der bei dem Einsatz in Gasphasenreaktionen üblichen schleichenden Desaktivierung, jedoch lassen sie sich vorteilhafterweise im Reaktor in Gegenwart von Luftsauerstoff thermisch vollständig regenerieren, vorzugsweise bei Temperaturen oberhalb von 300 °C, besonders bevorzugt bei Temperaturen oberhalb 400 °C, so dass ein regelmäßiger Betrieb des Reaktors ohne zeitund kostenintensiven Austausch des Katalysators gewährleistet ist.

Bei dem erfindungsgemäßen Verfahren können die Einsatzstoffe ohne Lösungsmittel oder zusammen mit dem Dampf eines Lösungsmittels über den sauren heterogenen Katalysator geleitet werden. Beispiele für geeignete Lösungsmittel sind Wasser, Alkohole wie Methanol, Ethanol, Isopropanol etc., Ether wie Tetrahydrofuran, Ethylenglykolmonomethylether, Ethylenglykoldimethylether etc., sowie Dimethylformamid und N-Methylpyrrolidinon. Bevorzugt wird das erfindungsgemäße Verfahren ohne Zusatz von Lösungsmitteln oder in Gegenwart von Wasser durchgeführt.

Das erfindungsgemäße Verfahren kann in einem weiten Bereich von Temperaturen und Drücken durchgeführt werden. Sie sind vom Fachmann so zu wählen, dass alle Reaktanten bzw. Produkte und das gegebenenfalls eingesetzte Lösungsmittel dampfförmig vorliegen und eine hinreichende Raum-Zeit-Ausbeute erreicht wird. Bevorzugt wird die erfindungsgemäße Eliminierungsreaktion in einem Temperaturbereich von 200 bis 500 °C, besonders bevorzugt im Bereich von 300 bis 450 °C, durchgeführt. Die bevorzugten Drücke liegen im Bereich von 10 mbar bis 5 bar, besonders bevorzugt ist die Ausführung bei Normaldruck.

Die für dieses Verfahren benötigten 2-Alkoxycycloalkanone (II) und 2-Hydroxycycloalkanonketale (III) lassen sich auf verschiedene Art und Weise herstellen, beispielsweise durch Ketalisierung des entsprechenden, gegebenenfalls aus einer Acyloinkondensation stammenden Hydroxycycloalkanons (X. Creary, A. J. Rollin, J. Org. Chem. 1977, 42, 4231).

Ein wichtiger Vorteil des erfindungsgemäßen Verfahrens besteht jedoch darin, dass die Einsatzstoffe der Formeln II und III leicht in technischen Mengen durch eine elektrochemische Reaktion zwischen dem entsprechenden Cycloalkanon und den entsprechenden Alkoholen hergestellt werden können. Ein Verfahren hierzu ist in der EP-A-460 451 offenbart, auf die hier in vollem Umfang Bezug genommen wird.

Falls gewünscht, kann das erfindungsgemäß erhaltene Produkt in üblicher Weise gereinigt werden, z. B. durch Destillation. Zweckmäßigerweise erfolgt eine fraktionierte Destillation. Je nach Siedepunkt des Produktes wird eine Vakuumdestillation vorgenommen. Im Allgemeinen erfolgt die Destillation bei einem Druck im Bereich von etwa 10 mbar bis 1 bar.

Es hat sich als vorteilhaft erwiesen, im einsatzmaterial enthaltenes Wasser bei der Destillation durch Zusatz eines geeigneten Azeotropbildners, z. B. Touluol, abzutrennen.

Vor der Destillation kann man gegebenenfalls vorhandene Säurespuren mit Basen, wie Alkalimetallhydroxide, -carbonate oder -bicarbonate, z. B. Natrium- oder Kaliumhydroxid, -carbonat oder -bicarbonat, neutralisieren.

Die folgenden Beispiele sollen das erfindungsgemäße Verfahren veranschaulichen, ohne jedoch den Umfang der Erfindung zu beschränken.

### Beispiele

### Beispiel 1

Als Katalysator wurde Siliciumdioxid verwendet, das mit 20 Gew.-% Phosphorsäure imprägniert war. Der Katalysator lag in 4 mm-Strängen vor. In einem vertikal angeordneten, elektrisch beheizten Rohrreaktor aus Quarzglas, in dem sich von unten nach oben 10 ml Quarzringe, dann der Katalysator (27,5 g) als Strangpreßlinge und anschließend nochmals 20 ml Quarzringe befanden, wurden bei einer Innentemperatur von 350 °C von oben 0,1 mol/h Stickstoff und 15 ml/h flüssiges 2-Hydroxycyclohexanondimethylketal dosiert. Die oben angeordnete Schicht aus Quarzringen diente als Verdampfer und bewirkte keine nennenswerte chemische Umsetzung des Einsatzstoffes. Die am unteren Ende austretenden Gase wurden einem mit Kühlwasser betriebenen Kondensator zugeführt. Das 2-Hydroxycyclohexanondimethylketal wurde zu > 99 % umgesetzt. Im Kondensat war 2-Cyclohexenon mit einer Ausbeute von etwa 63 % der Theorie enthalten. Das 2-Cyclohexenon wurde nach Destillation mit einer Reinheit > 99 % erhalten.

### Beispiel 2

Es wurde wie in Beispiel 1 verfahren, jedoch wurde anstelle von reinem 2-Hydroxycyclohexanondimethylketal ein 1:1-Gemisch desselben mit 2-Methoxycyclohexanon als Einsatzstoff verwendet. Im Kondensat war 2-Cyclohexenon zu 64 % enthalten.

## Patentansprüche

1. Verfahren zur Herstellung von 2-Cycloalkenonen der Formel I, 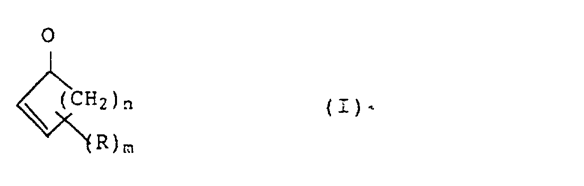 **dadurch gekennzeichnet, dass** man ein 2-Alkoxycycloalkanon der Formel II 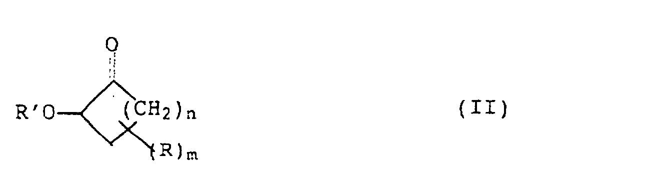 oder ein 2-Hydroxycycloalkanonketal der Formel III 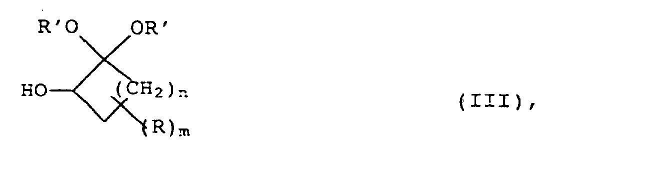 worin
n für eine ganze Zahl von 0 bis 20 steht,
m für 0, 1, 2 oder 3 steht,
R für Alkyl, Alkylaryl, Aryl, Arylalkyl, Alkenyl oder Alkinyl steht, wobei die Arylreste einen oder zwei Substituenten aufweisen können, die unabhängig voneinander ausgewählt sind unter Alkyl, Hydroxy oder Alkoxy,
die Reste R' unabhängig voneinander Alkyl oder Alkenyl bedeuten oder in Formel III gemeinsam für C₁-C₅-Alkylen oder C₂-C₅-Alkenylen stehen können,
oder ein Gemisch aus den Verbindungen der Formeln II und III an einem sauren heterogenen Katalysator einer Eliminierungsreaktion in der Gasphase unterwirft.

2. Verfahren nach Anspruch 1, **dadurch gekennzeichnet, dass** ein saurer heterogener Katalysator eingesetzt wird, der wenigstens ein Oxid oder Phosphat oder eine Mischung von Oxiden und Phosphaten oder ein Mischoxid von Elementen der Gruppen 3 bis 6 und 13 bis 15 des Periodensystems enthält.

3. Verfahren nach Anspruch 2, **dadurch gekennzeichnet dass** der saure heterogene Katalysator zusätzlich mit einer oder mehreren Mineralsäuren oder Metall- oder Ammoniumsalzen der Mineralsäuren imprägniert ist.

4. Verfahren nach Anspruch 2 oder 3, **dadurch gekennzeichnet, dass** man als sauren heterogenen Katalysator SiO₂, TiO₂, ZrO₂, Al₂O₃ oder LaPO₄ verwendet, der gegebenenfalls mit einer Mineralsäure oder einem Ammoniumsalz der Mineralsäure imprägniert ist.

5. Verfahren nach einem der Ansprüche 1 bis 4, **dadurch gekennzeichnet, dass** die Einsatzstoffe ohne Lösungsmittel oder zusammen mit dem Dampf eines Lösungsmittels über den sauren heterogenen Katalysator geleitet werden.

6. Verfahren nach einem der Ansprüche 1 bis 5, **dadurch gekennzeichnet, dass** man die Eliminierungsreaktion im Bereich von 200 °C bis 500 °C durchführt.

7. Verfahren nach einem der Ansprüche 1 bis 6, **dadurch gekennzeichnet, dass** man die Eliminierungsreaktion bei Drücken im Bereich von 10 mbar bis 5 bar durchführt.

8. Verfahren nach einem der Ansprüche 1 bis 7, **dadurch gekennzeichnet, dass** man die Verbindungen der Formeln II und III durch eine elektrochemische Reaktion zwischen dem entsprechenden Cycloalkanon und den entsprechenden Alkoholen herstellt.

9. Verfahren nach einem der Ansprüche 1 bis 8, **dadurch gekennzeichnet, dass** man das Verfahrensprodukt durch Zugabe einer Base neutralisiert.

10. Verfahren nach einem der Ansprüche 1 bis 9, **dadurch gekennzeichnet, dass** man das Verfahrensprodukt durch Destillation reinigt.

11. Verfahren nach Anspruch 10, **dadurch gekennzeichnet, dass** man während der Destillation noch vorhandenes Wasser abtrennt.

## Claims

1. A process for preparing 2-cycloalkenones of the formula I 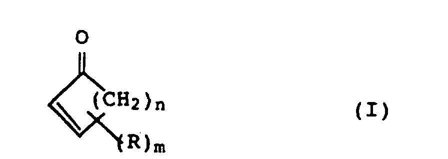 which comprises subjecting a 2-alkoxycycloalkanone of the formula II 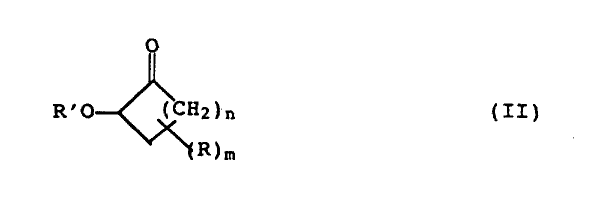 or a 2-hydroxycycloalkanone ketal of the formula III 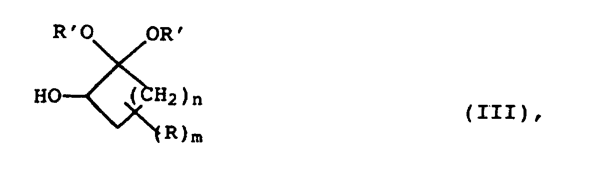 in which
n is an integer from 0 to 20,
m is 0, 1, 2 or 3,
R is alkyl, alkylaryl, aryl, arylalkyl, alkenyl or alkynyl, it being possible for the aryl radicals to have one or two substituents which are selected, independently of one another, from alkyl, hydroxyl or alkoxy,
the R' radicals are, independently of one another, alkyl or alkenyl or, in formula III, may together be C₁-C₅-alkylene or C₂-C₅-alkenylene,
or a mixture of the compounds of the formulae II and III to an elimination reaction in the gas phase on an acidic heterogeneous catalyst.

2. A process as claimed in claim 1, wherein an acidic heterogeneous catalyst which comprises at least one oxide or phosphate or a mixture of oxides and phosphates or a mixed oxide of elements of groups 3 to 6 and 13 to 15 of the Periodic Table is employed.

3. A process as claimed in claim 2, wherein the acidic heterogeneous catalyst is additionally impregnated with one or more mineral acids or metal or ammonium salts of the mineral acids.

4. A process as claimed in claim 2 or 3, wherein SiO₂, TiO₂, ZrO₂, Al₂O₃ or LaPO₄, which is, where appropriate, impregnated with a mineral acid or an ammonium salt of the mineral acid, is used as acidic heterogeneous catalyst.

5. A process as claimed in any of claims 1 to 4, wherein the starting materials are passed without solvent or together with the vapor of a solvent over the acidic heterogeneous catalyst.

6. A process as claimed in any of claims 1 to 5, wherein the elimination reaction is carried out in the range from 200°C to 500°C.

7. A process as claimed in any of claims 1 to 6, wherein the elimination reaction is carried out under pressures in the range from 10 mbar to 5 bar.

8. A process as claimed in any of claims 1 to 7, wherein the compounds of the formulae II and III are prepared by an electrochemical reaction between the appropriate cycloalkanone and the appropriate alcohols.

9. A process as claimed in any of claims 1 to 8, wherein the process product is neutralized by addition of a base.

10. A process as claimed in any of claims 1 to 9, wherein the process product is purified by distillation.

11. A process as claimed in claim 10, wherein water which is still present is removed during the distillation.

## Revendications

1. Procédé pour la fabrication de 2-cycloalcènones de formule I, 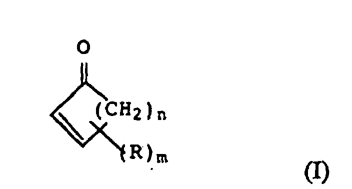 **caractérisé en ce que** l'on soumet un 2-alcoxycycloalcanone de formule **II** 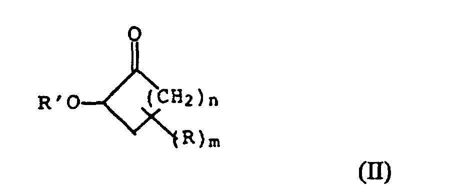 ou un 2-hydroxycycloalcanone-cétal de formule III 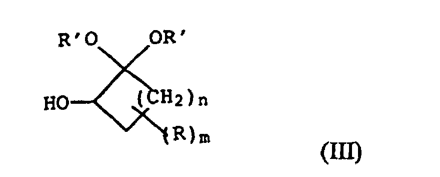 dans lesquelles
n représente un nombre entier de 0 à 20,
m représente 0, 1, 2 ou 3,
R représente alkyle, alkylaryle, aryle, arylalkyle, alkényle ou alkynyle, les résidus aryle pouvant présenter un ou deux substituants qui sont choisis indépendamment l'un de l'autre parmi alkyle, hydroxy ou alkoxy,
les résidus R' signifient, indépendamment l'un de l'autre, alkyle ou alkényle ou peuvent, dans la formule III, représenter conjointement C₁-C₅-alkylène ou C₂-C₅-alkylène,
ou un mélange des combinaisons des formules II et III, à une réaction d'élimination dans la phase gazeuse, sur un catalyseur acide hétérogène.

2. Procédé selon la revendication 1, **caractérisé en ce qu'**un catalyseur acide hétérogène, qui contient au moins un oxyde ou phosphate ou un mélange d'oxydes et de phosphates ou un oxyde mixte d'éléments des groupes 3 à 6 et 13 à 15 de la classification périodique, est utilisé.

3. Procédé selon la revendication 2, **caractérisé en ce que** le catalyseur acide hétérogène est, en plus, imprégné d'un ou de plusieurs acides minéraux ou sels de métaux ou d'ammonium des acides minéraux.

4. Procédé selon la revendication 2 ou 3, **caractérisé en ce que** l'on utilise comme catalyseur acide hétérogène SiO₂, TiO₂, ZrO₂, Al₂O₃ ou LaPO₄, qui est éventuellement imprimé d'un acide minéral ou d'un sel d'ammonium de l'acide minéral.

5. Procédé selon l'une des revendications 1 à 4, **caractérisé en ce que** les substances utilisées sont dirigées sur le catalyseur acide hétérogène sans solvant ou avec la vapeur d'un solvant.

6. Procédé selon l'une des revendications 1 à 5, **caractérisé en ce que** l'on réalise la réaction d'élimination dans la gamme de 200°C à 500°C.

7. Procédé selon l'une des revendications 1 à 6, **caractérisé en ce que** l'on réalise la réaction d'élimination à des pressions dans la gamme de 10 mbar à 5 bar.

8. Procédé selon l'une des revendications 1 à 7, **caractérisé en ce que** l'on produit les combinaisons des formules II et III par une réaction électrochimique entre la cycloalcanone adéquats et les alcools adéquats.

9. Procédé selon l'une des revendications 1 à 8, **caractérisé en ce que** l'on neutralise le produit du procédé par addition d'une base.

10. Procédé selon l'une des revendications 1 à 9, **caractérisé en ce que** l'on épure le produit du procédé par distillation.

11. Procédé selon la revendication 10, **caractérisé en ce que**, pendant la distillation, l'on sépare l'eau encore présente.
